# EUROPEAN PATENT APPLICATION

(11) **EP 3 620 156 A1**
(43) Date of publication of application: **11.03.2020**
(21) Application number: 18794991.2
(22) Date of filing: 30.04.2018
(51) Int. Cl.: A61K 9/20, A61K 9/48, A61K 31/44, A61K 9/00

(54) **COMPOSITION HAVING IMPROVED WATER SOLUBILITY AND BIOAVAILABILITY**

(30) Priority: 02.05.2017 KR 20170056115; 27.04.2018 KR 20180049305
(71) Applicant: Samyang Biopharmaceuticals Corporation, Seoul 03129 (KR)
(72) Inventor: PARK, Sang Yeob, Daejeon 34049 (KR); LIM, Hye Jung, Seongnam-si Gyeonggi-do 13490 (KR); LEE, Sa Won, Seongnam-si Gyeonggi-do 13554 (KR); SEO, Min Hyo, Yongin-si Gyeonggi-do 16827 (KR)
(74) Representative: Clarenbach, Carl-Philipp
(86) International application number: PCT/KR2018/004997
(87) International publication number: WO 2018/203636

(57) **Abstract**

Disclosed are: a composition having improved water solubility and bioavailability, containing sorafenib and a polymethacrylate copolymer; and a preparation method therefor.

## Description

### [Technical Field]

The present invention relates to an oral composition having improved water solubility and bioavailability, comprising a poorly water-soluble drug sorafenib and polymethacrylate copolymer, and a method for preparing the same.

### [Background Art]

Sorafenib (4-{4-[3-(4-chloro-3-trifluoromethylphenyl)-ureido]-phenoxy}-pyridine-2-carboxylic acid methyl amide) is a TKI (Tyrosine Kinase Inhibitor)-based target anticancer agent used for treating renal cell carcinoma, hepatocellular carcinoma and thyroid cancer, and has been released as an oral tablet at home and abroad.

Korean Patent Publication No. 1395932 discloses a pharmaceutical composition for treating a hyperproliferative disorder, including cancer, which is a tablet containing p-toluenesulfonic acid salt of sorafenib in an amount of at least 55% by weight of the composition, specifically a pharmaceutical composition comprising 3 to 20% of microcrystalline cellulose as filler, 5 to 12% of sodium croscarmellose as disintegrant, 0.5 to 8% of hypromellose as binder, 0.2 to 0.8% of magnesium stearate as lubricant and 0.1 to 2% sodium lauryl sulfate as a surfactant.

However, sorafenib has a very low solubility in water of 0.00171 mg/ml and the absolute bioavailability in humans is unknown. However, the relative bioavailability of tablets compared to oral solutions is considerably lower, ranging from 38% to 49%.

Various studies have been conducted to improve the low water solubility and bioavailability of sorafenib.

### [Detailed Description]

### [Technical Purpose]

The purpose of the present invention is to provide an oral composition having improved water solubility and bioavailability, comprising sorafenib and a polymethacrylate copolymer.

Another purpose of the present invention is to provide a method for preparing the oral composition having improved water solubility and bioavailability, comprising sorafenib and a polymethacrylate copolymer.

### [Technical Solution]

In order to achieve the technical purpose, the present invention provides an oral composition having improved water solubility and bioavailability, comprising sorafenib or a pharmaceutically acceptable salt thereof, and a polymethacrylate copolymer.

In another aspect, the present invention provides a method for preparing the oral composition having improved water solubility and bioavailability, comprising:
1) step of mixing sorafenib or a pharmaceutically acceptable salt thereof with a polymethacrylate copolymer; and
2) step of forming an oral formulation with the mixture of step 1).

### [Advantageous Effects]

Oral compositions comprising sorafenib and polymethacrylate copolymers according to the present invention can achieve the same effect in the body even when using relatively small amounts of drugs because the water solubility and bioavailability of sorafenib are improved. In addition, such a composition is known to be less affected by the diet, it may be very beneficial to the patient because there is a relatively small side effect of absorption due to fewer individual differences of absorption, and there may be an economic advantage due to less use of expensive drugs. That is, the water solubility and bioavailability of sorafenib are remarkably improved by polymethacrylate copolymer, so that even with reduced doses of drugs, formulations with equivalent bioavailability can be developed. Therefore, it is possible to provide a formulation that gives the patient an equivalent effect but reduces side effects and manufacturing costs.

### [Brief Description of the Figures]

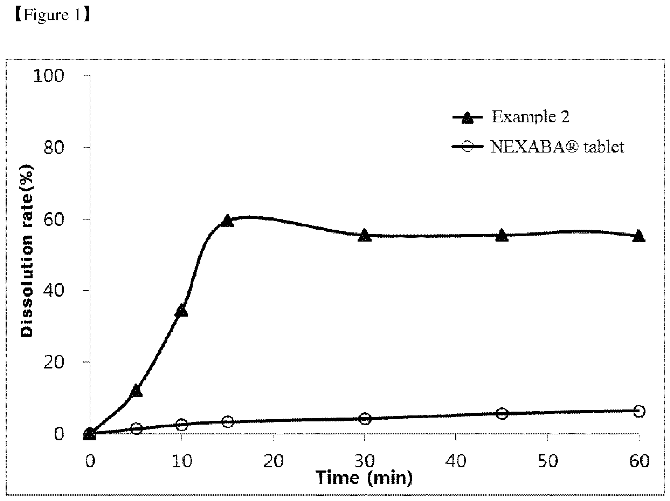

Fig. 1 is a graph showing the results of the dissolution test performed at pH 1.2 for the solid dispersion of Example 2 and Nexava® tablets (equivalent as 200 mg of sorafenib).

### [Mode for Invention]

### Definition of Terms

Unless otherwise expressly stated, some terms used throughout this specification may be defined as follows.

Unless specifically stated throughout this specification, "comprise" or "contain" refers to comprise any component without particular limitation, and it is not to be construed as excluding the addition of other components.

"Sorafenib" comprises sorafenib free base, isomers of drugs or mixtures thereof. "A pharmaceutically acceptable salt of sorafenib" comprises all types or forms of pharmaceutically acceptable salts with sorafenib as an active ingredient. In each case, it may be one that forms various hydrates, or various crystalline forms. For example, "a pharmaceutically acceptable salt of sorafenib" can be sorafenib tosylate, and can be amorphous, crystalline form 1, form 2, form 3 of sorafenib tosylate or mixtures thereof.

### Finding a solution

Various approaches have been used to improve water solubility and bioavailability of poorly water-soluble drugs. Modifications of the drug itself, such as a preparation of salts, crystalline forms and prodrugs, the production of micro- or nanoemulsions and solid dispersions, micronization or nanonization through the reduction of the particle size of the drug, micelle formation etc. have been mainly used. However, since each poorly water-soluble drug has different drug properties such as solubility, pH, pKa, melting point, molecular weight, functional group, conformational structure, crystallinity, permeability, absorption site, degradability, and effective drug dose, one technology cannot be applied to all drugs in common, and many efforts should be made to find a way that works well for a particular drug. In addition to the modification of the drug itself, it is very important to find a substance or combination of substances necessary for improving the water solubility and bioavailability of poorly water-soluble drugs.

Many attempts have been made to improve the water solubility and bioavailability of sorafenib, but no attempt has been made using polymethacrylate copolymers.

After devoting much effort to evaluate the effect of various materials on the water solubility of sorafenib, the present inventors have found a polymethacrylate copolymer having an excellent effect on improving the water solubility and bioavailability of sorafenib.

### Detailed description

The present invention provides an oral composition having improved water solubility and bioavailability, comprising sorafenib and a polymethacrylate copolymer, and a method for preparing the same.

Oral compositions of the present invention may preferably be in the form of solid oral formulations-in particular, tablets or capsules.

The sorafenib may be in various forms-in particular, in the form of micronized from several to several tens of micrometers. Preferably, it may be in micronized to several micrometers in size. More preferably, it may be in nanonized form to several hundred nanometers in size. As the particle size of the drug decreases, the dissolution rate may be improved to increase water solubility and bioavailability.

The polymethacrylate copolymer may be preferably a cationic polymer having dimethylaminoethyl methacrylate, more preferably poly (butylmethacrylate-co-(2-dimethylaminoethyl) methacrylate-co-methylmethacrylate).

The polymethacrylate copolymer may have a weight average molecular weight of 3,000 to 200,000 g/mole, preferably 5,000 to 150,000 g/mole, more preferably 10,000 to 100,000 g/mole, even more preferably 20,000 to 80,000 g/mole, most preferably 37,000 to 57,000 g/mole. If the weight average molecular weight is less than 3,000 g/mole, the water solubility improvement may be low. If the weight average molecular weight is more than 200,000 g/mole, disintegration may be delayed.

The state of the polymethacrylate copolymer is not particularly limited, but may be in a granular or powder state.

As a specific example, the polymethacrylate copolymer may be Eudragit® E PO (Evonik, Germany) or Eudragit® E 100 (Evonik, Germany). As a specific example, the weight average molecular weight of the polymethacrylate copolymer may be about 47,000 g/mole. Eudragit® E polymers are typically coating agents, and they are used for the purpose of relieving patient's discomfort with the drug and providing a smooth and shiny surface so that the medicine can be easily swallowed by encapsulating sensitive drugs or masking the taste and odor of a drug when it is necessary to protect the drug from moisture or the patient's medication compliance is poor. However, it is used as an additive to improve the water solubility and bioavailability of sorafenib in the present invention.

The polymethacrylate copolymer may be, for example, in an amount of 0.05 to 5 parts by weight, preferably 0.1 to 4 parts by weight, more preferably 0.2 to 3 parts by weight, even more preferably in an amount of 0.25 to 2 parts by weight based on 1 part by weight of sorafenib or a pharmaceutically acceptable salt thereof. If the amount of the polymethacrylate copolymer is less than the above lower limit, the improvement for water solubility and bioavailability of sorafenib may be insignificant, and the effect may be reduced. If the amount of the polymethacrylate copolymer is more than the above upper limit, the formulation (e.g., tablets) may become too large and cause discomfort when taken by the patient.

The present invention may comprise other additional components in addition to the sorafenib and polymethacrylate copolymers. Examples of other additional components include diluents, disintegrants, glidants and coating agents.

The diluents may be, for example, one or more selected from the group consisting of lactose (anhydrides or hydrates such as monohydrates), cellulose powder, microcrystalline cellulose, silicified microcrystalline cellulose, starch, dicalcium phosphate, tricalcium phosphate, magnesium trisilicate, mannitol, maltitol, sorbitol, xylitol, lactose, dextrose, maltose, sucrose, glucose, fructose, maltodextrin and mixtures thereof, but are not limited thereto. Preferably, lactose, microcrystalline cellulose or mixtures thereof may be selected. Most preferably, a mixture of starch, lactose and micelle crystalline cellulose can be selected. Diluents may also serve as binders.

The diluent may be used, for example, in an amount of 0.1 to 50 parts by weight, preferably in an amount of 1 to 30 parts by weight, more preferably 2 to 15 parts by weight, based on 100 parts by weight of the total formulation (e.g., tablets). If the amount of the diluent is less than the above lower limit, it may be difficult to prepare the formulation, such as low tableting. If the amount of the diluent is more than the above upper limit, the formulation becomes too large and may cause inconvenience when the patient takes it.

The disintegrants may be, for example, one or more selected from the group consisting of croscarmellose sodium (CrosCMC-Na), carboxymethylcellulose, crospovidone (crosslinked polyvinylpyrrolidone), L-HPC (low-substituted hydroxypropylcellulose), starch (wheat, rice, corn or potato starch), sodium carboxymethyl starch, sodium glycolate of potato starch, partially hydrolyzed starch and mixtures thereof, but are not limited thereto. Preferably, the disintegrants may be croscarmellose sodium (CrosCMC-Na), L-HPC (low-substituted hydroxypropyl cellulose) or a mixture thereof.

The disintegrant may be used, for example, in an amount of 1 to 30 parts by weight, preferably in an amount of 2 to 20 parts by weight, based on 100 parts by weight of the total formulation (e.g., tablets). If the amount of the disintegrant is less than the above lower limit, there may be a problem of dissolution rate delay due to the disintegration rate delay. If the amount of the disintegrant is more than the above upper limit, there may be a problem of productivity such as tableting disorder.

The glidants may be, for example, one or more selected from the group consisting of magnesium stearate, fumaric acid, stearic acid, calcium stearate, sodium stearyl fumarate, polyethylene glycol, starch (wheat, rice, corn or potato starch), talc, highly dispersed (colloidal) silica, magnesium oxide, magnesium carbonate, glyceryl behenate, glyceryl monostearate, silicon dioxide, calcium silicate, magnesium silicate and mixtures thereof, but are not limited thereto. Preferably, magnesium stearate may be selected.

The glidant may be used, for example, in an amount of 0.1 to 3 parts by weight, preferably in an amount of 0.2 to 3 parts by weight, more preferably in an amount of 0.5 to 2 parts by weight, based on 100 parts by weight of the total formulation (e.g., tablets). If the amount of the lubricant is less than the above lower limit, there may be a problem of productivity such as tableting disorder. If the amount of the lubricant is more than the above upper limit, there may be a problem of dissolution delay or productivity.

The coating agent may be a hydrophilic polymer and, for example, one or more selected from the group consisting of polyvinylpyrrolidone (PVP), polyvinylacetate (PVA), hydroxypropylmethylcellulose (HPMC), carboxymethylcellulose (sodium salt and calcium salt), ethyl cellulose, methylcellulose, hydroxyethylcellulose, ethylhydroxyethylcellulose, hydroxypropylcellulose (HPC), L-HPC (low-substituted HPC), polyvinyl alcohol, polymers of acrylic acid and salts thereof, vinylpyrrolidone-vinylacetate copolymers (e.g., Kollidon® VA64, BASF), polycoat IR, gelatin, guar gum, partially hydrolyzed starch, alginate, xanthan and mixtures thereof, but is not limited thereto. Preferably it may be polyvinylacetate (PVA), hydroxypropylmethylcellulose (HPMC) or polycoat IR.

The coating agent may be used, for example, in an amount of 0.2 to 10 parts by weight, preferably in an amount of 0.5 to 7 parts by weight, and more preferably in an amount of 1 to 5 parts by weight, based on 100 parts by weight of the tablet before coating (uncoated tablet). If the amount of the hydrophilic polymer is less than the above lower limit, there may be a problem in that a part of the entire surface of the uncoated tablet is not covered with the coating agent. If the amount of the hydrophilic polymer is more than the above upper limit, there may be a problem of excessive delay of the dissolution rate.

In the process of preparing the coated tablet as described above, various biologically inert ingredients may be used for additional purposes such as coating efficiency, drug stability, appearance, color, protection, retention, bonding, performance improvement and manufacturing process improvement.

According to one embodiment, the biologically inert component which may be further comprised in the coating layer may be one or more selected from the group consisting of a plasticizer, lubricant, colorant, flavoring agent, surfactant, stabilizer, antioxidant, foaming agent, antifoaming agent and desiccant (e.g., paraffin, wax). For example, the plasticizer may be 100% by weight or less (e.g., 0.1 to 100% by weight), specifically 50% by weight or less (e.g., 0.1 to 50% by weight), and more specifically 30% by weight or less (e.g., 0.1 to 30% by weight), based on the dry weight of the entire polymer used in each coating layer.

For example, the plasticizer may be one or more selected from the group consisting of triethyl citrate, dibutyl phthalate, diethyl phthalate, dibutyl sebacate, diethyl sebacate, tributyl citrate, acetyl triethyl citrate, acetyl triethyl citrate, propylene glycol, triacetin, polyethylene glycol, cetyl alcohol, stearyl alcohol and cetostearyl alcohol, but is not limited thereto.

The lubricant may be comprised in an amount of 100% by weight or less (e.g., 0.1 to 100% by weight) based on the dry weight of the entire polymer used for each coating layer.

Specifically, the glidants may be, for example, one or more selected from the group consisting of magnesium stearate, fumaric acid, stearic acid, calcium stearate, sodium stearyl fumarate, polyethylene glycol, starch (wheat, rice, corn or potato starch), talc, highly dispersed (colloidal) silica, magnesium oxide, magnesium carbonate, glyceryl behenate, glyceryl monostearate, silicon dioxide, calcium silicate, magnesium silicate and mixtures thereof, but is not limited thereto.

In the tablet or capsule, various additives may be mixed in order to improve physical properties, manufacturability, compressibility, appearance, taste and drug stability of the tablet or capsule. Such additives may include, for example, stabilizers, solubilizers, sweeteners, corrigents, pigments, wetting agents, fillers, stabilizers, surfactants, lubricants, solubilizers, buffers, sweeteners, adsorbents, corrigent, binders, suspending agents, hardeners, antioxidants, brighteners, fragrance ingredients, flavoring agents, pigments, coating agents, wetting agents, wetting regulators, fillers, defoamers, fresheners, chewing agents, antistatic agents, colorants, dragees, isotonic agents, emollients, emulsifiers, tackifiers, thickeners, foaming agents, pH adjusting agents, excipients, dispersants, disintegrants, waterproofing agents, preservatives, preserved agents, dissolution aids, solvents, glidants, and the like, but are not limited thereto, and any pharmaceutically acceptable agent may be used.

### Preparing method

Sorafenib and polymethacrylate copolymers can be formulated in a variety of ways in the preparation of oral compositions.

For example, sorafenib and polymethacrylate copolymers can be formulated by simple mixing. This mixture can be mixed with other additional ingredients to be tableted by tableting in a direct fashion or to be encapsulated by filling into capsules.

For example, sorafenib and other additional ingredients may be granulated in various ways, and then the polymethacrylate copolymer may be post-mixed and compressed into tablets, or they may be filled into capsules and encapsulated. Examples of granulation in various ways include wet granulation, dry granulation and the like. Wet granulation can be used with high speed mixers or fluidized bed granulators, and dry granulation can be used with roller compactors and extruders.

For example, the sorafenib and polymethacrylate copolymers can be mixed together, granulated in various ways and tableted by post-mixing other additional ingredients or encapsulated by filling into capsules.

For example, in order to granulate sorafenib, the polymethacrylate copolymer may be dissolved in a solvent such as ethanol and used as a binder to prepare wet granules, and then post-mixed with other additional components to be tableted or filled into capsules to be encapsulated.

For example, the sorafenib and polymethacrylate copolymers may be mixed, melt-extruded into pellets at appropriate temperature conditions using a melt extruder and then filled into capsules or mixed with other additional ingredients to be tableted.

For example, the sorafenib and polymethacrylate copolymer may be formulated into a solid dispersion obtained by dissolving them in an appropriate solvent and then removing the solvent by spray drying or the like. Such solid dispersions can be mixed with other additional ingredients to be tableted, or encapsulated by filling in a capsule.

The present invention will be described in more detail through the following Examples. However, these Examples are only intended to describe the present invention exemplarily, and the scope of the present invention is not at all limited by them.

### <Test Example 1: Content Test>

In order to test the content of drug included in the formulation, it was analyzed by HPLC using the following test method.

### 1) Preparation of mobile phase solution

(1) Buffer preparation: After dissolving 0.77 g ammonium acetate in 1000 ml of water, the pH of the solution was adjusted to 6.8 ± 0.05 using ammonia solutions.
(2) The solution of (1) and acetonitrile were mixed at 40:60 (v/v), and the solution filtered with a filter paper having a pore size of 0.45 µm and degassed was used as a mobile phase.

### 2) Preparation of diluent

The buffer solution (1) and acetonitrile were mixed at 30:70 (v/v) to obtain a dilution solution.

### 3) Preparation of standard solution

30 mg of sorafenib tosylate standard was precisely taken into a 100 ml flask to dissolve well with diluent, and the solution which matched the mark was used as the standard liquid (300 µg/ml). 1 ml of the standard solution (300 µg/ml) was precisely taken, and 2 ml of the diluted solution was added to prepare a standard solution (100 µg/ml). 1 ml of the standard solution (300 µg/ml) was precisely taken, and 4 ml of the diluted solution was added to prepare a standard solution (60 µg/ml). 1 ml of the standard solution (100 µg/ml) was precisely taken, and 4 ml of the diluted solution was added to prepare a standard solution (20 µg/ml). A calibration curve was drawn using the four standard solutions and used to calculate the content of the test solution.

### 4) Preparation of test liquid

An appropriate concentration was made using a volumetric flask and a diluent to dissolve and dilute sorafenib tosylate in the contents well, and the solution filtered through a syringe filter having a pore size of 0.45 µm was used as a test liquid.

### 5) Operation and calculation

The standard solution and the test liquid were injected into the column at appropriate time intervals under the following conditions, and the peak area was calculated to calculate the content of sorafenib tosylate (%) in the tablet.

### [Operation Conditions]

Detector: ultraviolet absorbance photometer (wavelength 265 nm)
Column: Eclipse XDB-C18, 4.6 mm × 150 mm, 5 µm or equivalent column
Flow rate: 1.0 ml/min
Injection volume: 20 µl
Column temperature: 27°C

### <Example 1: Additive screening>

In order to find an additive that helps increase the water solubility of sorafenib, 100 mg of sorafenib tosylate (crystalline form III) was added to the microtube, 10 mg of the additive was added, and 1 ml of pH 1.2 buffer was added. It was shaken at 1,100 rpm for 24 hours. Thereafter, the mixture was filtered with a syringe filter having a pore size of 0.45 µm, and as a result of performing content analysis according to the method of Test Example 1 by HPLC, the amount of dissolved drug was confirmed as follows.

**[Table 1]**

| Additive name | Concentration of dissolved sorafenib (µg/Mℓ) |
|---|---|
| No polymer | 0.1 |
| HPMC 2910 | 9.4 |
| HPMC 2208 | 8.4 |
| Kollidone K 30 | 46.3 |
| Kollidone VA64 | 28.3 |
| Soluplus | 11.7 |
| Pluronic F127 | 45.6 |
| Pluronic F68 | 1.5 |
| **EUDRAGIT E PO** | **395.5** |
| POLYOX WSR N10 (Polyethylene oxide) | 0.2 |
| HPC (Hydroxy propyl cellulose) | 1.7 |
| SDS (Sodium dodecyl sulphate) | 0.0 |
| Cremophor RH 40 | 79.3 |
| Tween 20 | 66.0 |
| Tween 80 | 101.3 |
| Labrafil M2130CS | 0.4 |
| Gelucire 44/14 | 35.2 |
| Eudragit RS | 0.1 |
| Eudragit S | 0.1 |
| Eudragit L | 0.1 |

### <Example 2: Preparation of solid dispersion>

2.67 g of sorafenib tosylate (crystalline Form III) and 5.34 g of Eudragit E 100 were dissolved in 150 ml of EtOH (37°C), and then distilled under reduced pressure using a rotary evaporator. The solvent was completely removed using a vacuum pump. Solid dispersions of sorafenib tosylate and Eudragit E formed on the wall were obtained. Yield 65%.

### <Example 3: Preparation of solid dispersion>

2.67 g of sorafenib tosylate (crystalline form III) and 5.34 g of Eudragit E 100 were dissolved in 150 ml of EtOH (37°C), followed by spray drying to remove the solvent, and the formed solid dispersion of sorafenib tosylate and Eudragit E were obtained. Yield 73%.

### <Example 4: Preparation of tablets>

274 g of micronized sorafenib tosylate (crystalline form III, average 2 µm) and 137 g of Eudragit E PO were mixed, followed by mixing with 30 g of microcrystalline cellulose, 45 g of croscarmellose and 4.5 g of magnesium stearate. The tablets were compressed using a single tablet press equipped with punch having a long axis 16.4 mm and a short axis 6.4 mm to obtain tablets with improved water solubility and bioavailability.

### <Test Example 2: Dissolution test>

For the solid dispersion obtained in Example 2 and the control drug NEXABA® tablets, the dissolution test was performed in 900 ml of a pH 1.2 buffer solution at 37°C at 50 rpm with the paddle method of the dissolution test method according to the Korean Pharmacopoeia General Test (equivalent as 200 mg of sorafenib). The analysis was the same as the HPLC analysis of Test Example 1, but the concentration of the standard solution was prepared by measuring 304 µg/ml.

The results are shown in Fig. 1. It can be seen that the solid dispersion of Example 2 was significantly improved in dissolution compared to the control drug.

## Claims

1. An oral composition having improved water solubility and bioavailability, comprising sorafenib or a pharmaceutically acceptable salt thereof, and a polymethacrylate copolymer.

2. The composition according to claim 1, wherein the polymethacrylate copolymer is a cationic polymer having dimethylaminoethyl methacrylate.

3. The composition according to claim 2, wherein the polymethacrylate copolymer is poly (butylmethacrylate-co-(2-dimethylaminoethyl) methacrylate-co-methylmethacrylate).

4. The composition according to claim 1, wherein the polymethacrylate copolymer has a weight average molecular weight of 3,000 to 200,000 g/mole.

5. The composition according to claim 1, wherein the polymethacrylate copolymer is in a granular or powder state.

6. The composition according to claim 1, comprising 0.05 to 5 parts by weight of polymethacrylate copolymer based on 1 part by weight of sorafenib.

7. The composition according to any of claims 1 to 6, wherein the pharmaceutically acceptable salt of sorafenib is sorafenib tosylate.

8. The composition according to any of claims 1 to 6, which is in the form of a solid oral formulation.

9. A method for preparing the oral composition having improved water solubility and bioavailability according to any of claims 1 to 6, comprising:
1) step of mixing sorafenib or a pharmaceutically acceptable salt thereof with a polymethacrylate copolymer; and
2) step of forming an oral formulation with the mixture of step 1).
